# EUROPEAN PATENT SPECIFICATION

(11) **EP 0 461 134 B1**
(45) Date of publication and mention of the grant of the patent: **17.05.2000**
(21) Application number: 90903390.4
(22) Date of filing: 22.02.1990
(51) Int. Cl.: A01N 43/30, A61K 7/40

(54) **PEDICULICIDAL COMPOSITIONS**
PEDICULIZIDE ZUSAMMENSETZUNGEN
COMPOSITIONS PEDICULICIDES

(30) Priority: 28.02.1989 GB 8904541
(43) Date of publication of application: 18.12.1991
(73) Proprietor: UNICLIFFE LIMITED, Stonar, Kent CT13 9NJ (GB)
(72) Inventor: OLIVER, William, John, Hampshire GU17 0BF (GB)
(74) Representative: Woodcraft, David Charles
(86) International application number: GB9000303
(87) International publication number: WO9009738

(56) References cited:
- EP-A- 0 143 297
- The Merck Index, Tenth Edition, 1983, Merck & Co., Inc., (Rahway, N.J., US), see page 1078, compound No. 7350
- Chemical Patents Index, Basic Abstracts Journal, Section C, week B26, 22 August 1979, Derwent Publications Ltd, (London, GB), see pages 53 Abstract 4804l & JP-A-54062316 (Asahi Chemical Ind K.K.)*

## Description

The present invention relates to pediculicidal compositions and includes a method of controlling louse infestations.

Head lice are a common health problem and spread rapidly in institutions such as the Armed Services and in schools. One of the difficulties in controlling head lice is that the eggs attach themselves very firmly to the base of hairs by adhesive secretions which makes them difficult to remove. Second, the eggs are protected by a waxy cuticle which is not penetrated by currently available anti-louse preparations. Conventional methods of controlling head lice therefore depend on the use of a medicated shampoo or lotion which loosens the natural adhesive or gum which binds the eggs to the hair of the scalp and at the same time is toxic to the hatched and active lice.

Although many of these conventional preparations are satisfactory in some cases if used conscientiously, their success does depend on very thorough repetitive treatments over a long period. They also have the disadvantage that many of the louse repellants which are used have unpleasant smells and this discourages their proper and continuous use over the required period of treatment.

We have now discovered that a compound which is related to benzaldehyde is very effective against louse eggs and also acts as a louse repellant.

According to the invention there is provided a method of preparing a lice repellent composition by forming a solution containing 1-10 wt% piperonal and a crystallization suppressing component selected from alcohols higher than propyl, terpenes and/or water.

According to the invention there is also provided a method of preparing a pediculicidal and lice repellent composition for controlling head lice which method comprises forming a solution of between 1 and 10wt% piperonal and 0.3 to 3% of a film forming polymer substantive to the hair of the lice's host selected from a vinyl pyrrolidone polymer, a vinyl acetate polymer, a quaternized collagen, a quaternized cellulose derivative or a polymer containing hydroxy and amino groups.

Piperonal is an aromatic aldehyde, and has the formula: 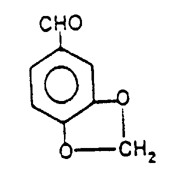 and its preparation is given in U.S. Patent No. 2916499 and J. Org. Chem. 26, 4814 (1961).

In the past, piperonal has been used quite widely as a constituent of perfumes and flavours, at relatively low concentrations, e.g. 50 ppm, giving a sweet floral tone to fragrances.

Piperonal is soluble in alcohols, glycols and some oils.

Moore (J. Am. Med. Ass., Vol. 71, p.530-531 (1918)) discloses pediculicidal compositions for the impregnation of underwear comprising piperonal (heliotropin) in oil or ether with fat or wax.

In the practice of the present invention, it is fairly important that the piperonal is maintained in solution since it is not as effective in its solid state. Although piperonal is soluble in lower alcohols, e.g. ethanol or isopropanol, the alcohol tends to separate leaving crystalline piperonal deposited on the body or hairs of the host. It has been found that a long-lasting repellent action is achieved by incorporating a hydrophilic film-forming component with the composition. Although not fully established, it is believed that the hydrophilic film-forming component reduces the rate of evaporation of the solvent, thus preventing crystallisation. Whatever the precise mechanism of this advantageous effect, formulations which contain the film-forming component have been shown to exhibit a strong repellent effect towards lice which lasts for up to 6 to 8 hours after the application to the hair.

Solutions of piperonal containing about 1% of the active ingredient are generally not very active. A concentration of at least about 3% is generally desirable and we prefer compositions containing at least 4% of piperonal. There is no critical upper limit but there is usually no advantage in using more that about 10%.

As indicated above, piperonal is soluble in alcohols, glycols and some oils, particularly terpene oils. Ethanol or propanol is conveniently used as the main solvent. However, in order to reduce its rate of evaporation, alcohols higher than propyl, glycols, terpenes and/or water may be included in the formulation. For example, the solvent may comprise 50 to 70% ethanol or propanol. Terpenes are a preferred optional ingredient, particularly if maximum ovicidal properties are desired. It is a property of terpenes that they enhance the penetration by the piperonal of the waxy cuticle of the lice eggs. Another desirable optional component is an aliphatic long chain hydrocarbon, which appears to enhance the wetting of the skin of the lice with the pediculicidal compositions.

The following Examples in which all percentages are by weight give illustrative pediculicidal compositions in accordance with the invention.

The ingredients specified in the following Examples were mixed in the amounts stated to give the corresponding formulations.

### Example 1

| Piperonal Lotion with terpenes and long chain alkanes | |
|---|---|
| Piperonal | 5.0% w/w |
| Long chain alkane mixture (Shellsol T) | 5.0% w/w |
| Terpineol | 8.2% w/w |
| d-Limonene | 5.3% w/w |
| iso Propyl Alcohol | 76.5% w/w |
| Shellsol T is a complex mixture of long chain alkanes. Terpineol and d-Limonene are naturally occurring terpenes with fairly high boiling points. | |

### Example 2

| Piperonal Lotion with Essential Oil and Copolymeric Resin | |
|---|---|
| Piperonal | 5.0% w/w |
| Thyme Oil | 0.5% w/w |
| Isopropyl myristate | 2.0% w/w |
| Propylene Glycol | 10.0% w/w |
| Adipic acid copolymer resin (Cartaretin F) | 1.0% w/w |
| iso Propyl Alcohol | 50.0% w/w |
| Water | 31.5% w/w |
| Thyme oil is a volatile oil distilled from THYMUS VULGARIS comprises mainly of Thymols and carvacrol. It is a counter-irritant. Cartaretin F is an Adipic acid/Dimethylamino hydroxy propyl diethylene triamine copolymer. It is a hair fixative. | |

### Example 3

| Piperonal Lotion with Soluble Lanolin | |
|---|---|
| Piperonal | 5.0% w/w |
| Soluble Lanolin (Lanexol AWS) | 2.0% w/w |
| Ethanol | 60.0% w/w |
| Water | 33.0% w/w |
| Soluble lanolin is a modified lanolin. Lanolin is a complex natural wax comprising of esters and polyesters of long chain fatty acids and fatty alcohols. | |

### Example 4

| Piperonal Lotion with PVP/VA Copolymer Resin | |
|---|---|
| Piperonal | 5.0% w/w |
| PVP/VA resin (Kollidon VA 64) | 1.0 w/w |
| Ethanol | 60.0% w/w |
| Water | 34.0% w/w |
| PVP/VA is a polyvinyl pyrrolidone/vinyl acetate copolymer resin. It is a water soluble film former. | |

### Example 5

| Piperonal Lotion with Polymer Resin | |
|---|---|
| Piperonal | 5.0% w/w |
| PVP (Kollidon 90) | 1.0% w/w |
| Ethanol | 60.0% w/w |
| PVP is a polyvinyl pyrrolidone polymeric resin. It is a water soluble film former. | |

### Example 6

| Piperonal Lotion with Quaternized Collagen Protein | |
|---|---|
| Piperonal | 5.0% w/w |
| Quarternized Collagen Protein (Crotein 0) | 0.5% w/w |
| Ethanol | 60.0% w/w |
| Water | 34.5% w/w |
| Quaternized Collagen protein is a quarternary ammonium derivative of hydrolysed collagen protein. It is a water soluble, highly substantive conditioner. | |

### Example 7

| Piperonal Lotion with Modified Silicone Fluid | |
|---|---|
| Piperonal | 5.0% w/w |
| Modified Silicone Fluid (Dow Corning 190) | 1.0% w/w |
| Ethanol | 60.0% w/w |
| Water | 34.0% w/w |
| Modified silicone fluid (Dow Corning 190) is a Silicone glycol copolymer. It is a wetting and spreading agent. | |

### Example 8

| Piperonal Lotion with Quaternized Cellulose | |
|---|---|
| Piperonal | 5.0% w/w |
| Quarternized cellulose (CrodaCel QL) | 1.0% w/w |
| Ethanol | 60.0% w/w |
| Water | 34.0% w/w |
| Quaternized cellulose derivatives are produced by quaternized of hydroalkyl celluloses with a range of alkyl groups. They are conditioning aids. | |

Other pediculicidal compounds may be included in the compositions of this invention, e.g. malathion or carbaryl sevin, although this is not essential. It is believed that piperonal acts by penetrating the waxy cuticle of the louse eggs and inactivating them and thus does not depend on the dislodging of the eggs from hair strands as do many conventional anti-louse preparations.

In the treatment of head lice the piperonal containing formulation is applied to the hair and left in contact for periods between two to twelve hours. Other additives may be included in the preparation, e.g. surfactants to increase the wetting of the hair and ingredients to depress the rate of evaporation of the solution.

The compositions of the present invention have several advantages over conventional anti-louse compositions. In addition to their effectiveness in killing adult lice and lice eggs, they exhibit superior repellent activity against adult lice. Moreover, because piperonal has a pleasant smell and is approved by government licensing authorities as a food additive, there is no danger of any health risk in using the compositions nor any antipathy among infected persons against acceptance of treatment.

While the invention has been described with particular reference to combating head lice infestations, the compositions of the invention are also effective in the same way against other lice, e.g. crab lice, clothing lice and body lice.

## Claims (Claims for the following Contracting State(s): AT, BE, CH, LI, DE, DK, FR, GB, IT, LU, NL, SE.)

1. A lice repellent composition for controlling head lice which comprises a solution of piperonal, said solution also containing a component selected from alcohols higher than propyl, terpenes and/or water which maintains the piperonal in solution thus suppressing crystallisation, and maintaining the solution in contact with the host for at least 2 hours, the piperonal being present in the solution in an amount between 1 and 10% by weight, the composition being capable of wetting the hair of the person to be treated.

2. A composition according to claim 1 which contains at least 3% by weight of piperonal.

3. A composition according to claim 2 which contains from 4 to 10% by weight of piperonal.

4. A pediculicidal and lice repellent composition for controlling head lice which comprises a solution of between 1 and 10% by weight of piperonal and from 0.3 to 3% of a film-forming polymer which is substantive to the hair of the host and is capable of maintaining the piperonal in solution in contact with the hair of a host for at least 2 hours, said film-forming polymer being selected from a vinyl pyrrolidone polymer, a vinyl acetate polymer, a quaternized collagen, a quaternized cellulose derivative or a polymer containing hydroxy and amino groups.

5. A composition according to claim 4 in which the film-forming polymer is a hydrophilic polymer.

6. Use of a piperonal solution as claimed in any one of claims 1 to 5 as a lice repellent.

## Claims (Claims for the following Contracting State(s): ES)

1. A method of preparing a lice repellent composition for controlling head lice by forming a solution containing 1-10 wt% piperonal and a crystallisation suppressing component selected from alcohols higher than propyl, terpenes and/or water, the composition being capable of wetting the hair of the person to be treated

2. A method as claimed in claim 1 wherein the solution contains at least 3wt% piperonal.

3. A method as claimed in claim 2 wherein the solution contains 4 to 10wt% piperonal.

4. A method of preparing a pediculicidal and lice repellent composition for controlling head lice which method comprises forming a solution of between 1 and 10wt% piperonal and 0.3 to 3% of a film forming polymer substantive to the hair of the lice's host selected from a vinyl pyrrolidone polymer, a vinyl acetate polymer, a quaternized collagen, a quaternized cellulose derivative or a polymer containing hydroxy and amino

5. A method as claimed in claim 4 in which the film-forming polymer is a hydrophilic polymer.

## Patentansprüche (Patentansprüche für folgende(n) Vertragsstaat(en): AT, BE, CH, LI, DE, DK, FR, GB, IT, LU, NL, SE.)

1. Lausabwehrmittel-Zusammensetzung zur Bekämpfung von Kopfläusen, umfassend eine Lösung von Piperonal, wobei diese Lösung auch einen Bestandteil ausgewählt aus Alkoholen höher als Propyl, Terpenen und/oder Wasser enthält, der das Piperonal in Lösung hält und damit die Kristallisation unterdrückt, und der die Lösung mindestens 2 Stunden in Kontakt mit dem Wirt hält, wobei das Piperonal in der Lösung in einer Menge zwischen 1 und 10 Gewichtsprozent enthalten ist und die Zusammensetzung geeignet ist, das Haar der zu behandelnden Person zu benetzen.

2. Zusammensetzung nach Anspruch 1, die mindestens 3 Gewichtsprozent Piperonal enthält.

3. Zusammensetzung nach Anspruch 2, die von 4 bis 10 Gewichtsprozent Piperonal enthält.

4. Pediculicidal- und Lausabwehrmittel-Zusammensetzung zur Bekämpfung von Kopfläusen, umfassend eine Lösung von zwischen 1 und 10 Gewichtsprozent Piperonal und von 0,3 bis 3% eines filmbildenden Polymers, welches für das Haar des Wirts substantiv ist und geeignet ist, das Piperonal in der Lösung mindestens 2 Stunden lang in Kontakt mit dem Haar eines Wirts zu halten, wobei das filmbildende Polymer ausgewählt wird aus einem Vinylpyrrolidonpolymer, einem Vinylacetatpolymer, einem quaternisierten Kollagen, einem quaternisierten Zellulosederivat oder einem Polymer, welches Hydroxy- und Aminogruppen enthält.

5. Zusammensetzung nach Anspruch 4, in der das flimbildende Polymer ein hydrophiles Polymer ist.

6. Verwendung einer Piperonal-Lösung nach einem der Ansprüche 1 bis 5 als Lausabwehrmittel.

## Patentansprüche (Patentansprüche für folgende(n) Vertragsstaat(en): ES)

1. Verfahren zur Herstellung einer Lausabwehrmittel-Zusammensetzung zur Bekämpfung von Kopfläusen mittels Bildung einer Lösung enthaltend 1-10 Gewichtsprozent Piperonal und einen kristallisationshemmenden Bestandteil, der ausgewählt ist aus Alkoholen höher als Propyl, Terpenen und/oder Wasser, wobei die Zusammensetzung geeignet ist, das Haar der zu behandelnden Person zu benetzen.

2. Verfahren nach Anspruch 1, wobei die Lösung mindestens 3 Gewichtsprozent Piperonal enthält.

3. Verfahren nach Anspruch 2, wobei die Lösung von 4 bis 10 Gewichtsprozent Piperonal enthält.

4. Verfahren zur Herstellung einer Pediculicidal- und Lausabwehrmittel-Zusammensetzung zur Bekämpfung von Kopfläusen, umfassend die Bildung einer Lösung von zwischen 1 und 10 Gewichtsprozent Piperonal und von 0,3 bis 3% eines filmbildenden Polymers, welches für das Haar des Lauswirts substantiv ist und ausgewählt ist aus einem Vinylpyrrolidonpolymer, einem Vinylacetatpolymer, einem quaternisierten Kollagen, einem quaternisierten Zellulosederivat oder einem Polymer, welches Hydroxy- und Aminogruppen enthält.

5. Verfahren nach Anspruch 4, bei dem das filmbildende Polymer ein hydrophiles Polymer ist.

## Revendications (Revendications pour l'(les) Etat(s) contractant(s) suivant(s): AT, BE, CH, LI, DE, DK, FR, GB, IT, LU, NL, SE.)

1. Composition répulsive anti-pou destinée à éliminer les poux de la tête, qui comprend une solution de pipéronal, ladite solution contenant également un constituant choisi parmi des alcools plus longs que le propanol, des terpènes et/ou de l'eau qui garde le pipéronal en solution supprimant ainsi une cristallisation, et maintenant la solution en contact avec l'hôte pendant au moins 2 heures, le pipéronal étant présent dans la solution en une quantité entre 1 et 10 % en poids, la composition étant capable de mouiller les cheveux de la personne se trouvant traitée.

2. Composition selon la revendication 1, qui contient au moins 3 % en poids de pipéronal.

3. Composition selon la revendication 2, qui contient de 4 à 10 % en poids de pipéronal.

4. Composition répulsive anti-pou et anti-pédiculose destinée à éliminer les poux de la tête, qui comprend une solution de pipéronal entre 1 et 10 % en poids et de 0,3 jusqu'à 3 % d'un polymère filmogène qui est relatif aux cheveux d'un hôte et qui est capable de garder le pipéronal en solution en contact avec les cheveux de l'hôte pendant au moins 2 heures, ledit polymère filmogène étant choisi parmi un polymère de vinylpyrrolidone, un polymère acétate de vinyle, un collagène quaternarisé, un dérivé quaternarisé de cellulose, ou un polymère contenant des groupements hydroxyle et amino.

5. Composition selon la revendication 4, dans lequel le polymère filmogène est un polymère hydrophile.

6. Utilisation d'une solution de pipéronal selon l'une des revendications 1 à 5, comme répulsif contre les poux.

## Revendications (Revendications pour l'(les) Etat(s) contractant(s) suivant(s): ES)

1. Procédé pour préparer une composition répulsive anti-pou destinée à éliminer les poux de la tête, en formant une solution contenant 1 - 10 % en poids de pipéronal et un constituant supprimant une cristallisation choisi parmi des alcools plus long que le propanol, des terpènes et/ou de l'eau, la composition étant capable de mouiller les cheveux de la personne se trouvant traiter.

2. Procédé selon la revendication 1, dans lequel la solution contient au moins 3 % en poids de pipéronal.

3. Procédé selon la revendication 1, dans lequel la solution contient de 4 jusqu'à 10 % en poids de pipéronal.

4. Procédé pour préparer une composition répulsive anti-pou et anti-pédiculose destinée à éliminer les poux de la tête, lequel procédé comprend les étapes consistant à former une solution entre 1 et 10 % en poids de pipéronal et de 0,3 à 3 % d'un polymère filmogène relatif aux cheveux de l'hôte du pou choisi parmi un polymère de vinylpyrrolidone, un polymère acétate de vinyle, un collagène quaternarisé, un dérivé quaternarisé de cellulose ou un polymère contenant des groupements hydroxyle et amino.

5. Procédé selon la revendication 4, dans lequel le polymère filmogène est un polymère hydrophile.
